(19) Europäisches Patentamt
European Patent Office
Office européen des brevets

(11) Veröffentlichungsnummer: **0 061 654**
**B1**

# (12) EUROPÄISCHE PATENTSCHRIFT

(45) Veröffentlichungstag der Patentschrift: **10.07.85**

(51) Int. Cl.⁴: **C 07 D 461/00**, C 07 C 143/86, A 61 K 31/475

(21) Anmeldenummer: **82102180.5**

(22) Anmeldetag: **17.03.82**

## (54) Vincamin-cyclamat, Verfahren zu seiner Herstellung und dieses enthaltende Arzneimittel.

(30) Priorität: **01.04.81 DE 3113132**

(43) Veröffentlichungstag der Anmeldung: **06.10.82 Patentblatt 82/40**

(45) Bekanntmachung des Hinweises auf die Patenterteilung: **10.07.85 Patentblatt 85/28**

(84) Benannte Vertragsstaaten: **AT BE CH DE FR GB IT LI LU NL**

(56) Entgegenhaltungen:
**DE - A - 2 234 329**
**DE - A - 2 500 599**
**DE - A - 2 721 171**
**DE - A - 2 725 246**

**ULLMANNS ENCYKLOPÄDIE DER TECHNISCHEN CHEMIE, 3. Auflage, 1965, 16. Band**

**Die Akte enthält technische Angaben, die nach dem Eingang der Anmeldung eingereicht wurden und die nicht in dieser Patentschrift enthalten sind.**

(73) Patentinhaber: **Heinrich Mack Nachf., Postfach 140, D-7918 Illertissen (DE)**

(72) Erfinder: **Stoss, Peter, Dr., Mozartstrasse 15, D-7918 Illertissen (DE)**

(74) Vertreter: **Lederer, Franz, Dr. et al, Patentanwälte Dr. Franz Lederer Dipl.-Ing. Reiner F. Meyer-Roxlau Lucile-Grahn-Strasse 22, D-8000 München 80 (DE)**

EP 0 061 654 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein neues Vincamin-Derivat, nämlich Vincamin-cyclamat der Formel I, dessen Herstellung sowie diese neue Verbindung enthaltende Arzneimittel.

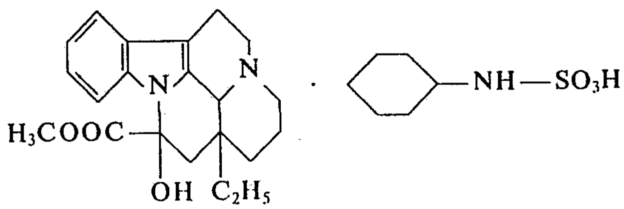

(I)

Vincamin ist ein aus Vinca minor L. isoliertes Alkaloid, das bekanntlich pharmakologische Aktivität als gefäßerweiterndes Mittel besitzt und zur Behandlung von Durchblutungsstörungen des Gehirns und neurologischen Störungen verwendet wird. Vincamin-Base ist in Wasser und anderen pharmazeutisch annehmbaren Lösungsmitteln praktisch unlöslich und wird hauptsächlich oral in festen Dosierungsformen, wie Tabletten oder Dragees verabreicht. Vincamin steht auch als Hydrochlorid in fester Form in Ampullen zur Zubereitung verdünntwäßriger Lösungen mit weniger als etwa 0,5 Gew./Gew.-% oder von Suspensionen zur Injektionsverabreichung unter Rekonstitution mit Wasser zur Verfügung.

Ein besonderes Problem bestand darin, eine Vincamin-Form, als Salz oder anderes Derivat zu finden, die in einem pharmazeutisch annehmbaren Lösungsmittel hinreichend löslich ist, um eine ausreichende Konzentration des Wirkstoffs in der Lösung zu liefern, so daß die Verabreichung in gelöster Form, vorzugsweise als Tropfen, möglich wird, d. h., die nötige Dosierungsmenge des Wirkstoffs in einem annehmbar kleinen Lösungsmittelvolumen zu ermöglichen. Ein weiteres, beträchtliches Problem liegt in der Tatsache, daß selbst die wenigen Salze oder Komplexe des Vincamins, die sich in pharmazeutisch annehmbaren Lösungsmittel als einigermaßen löslich erwiesen haben, z. B. Vincamin-citrat oder tartrat, und die daher eine brauchbare Konzentration des Wirkstoffs in Lösung liefern würden, einen extrem bitteren und unangenehmen Geschmack haben, der unmöglich zu maskieren oder zu überwinden ist, auch nicht durch Zusatz von Geschmacks- oder Aromastoffen. Die bisher vergeblichen Bemühungen, eine Form des Vincamins zu finden, die sowohl die gewünschte Löslichkeit als auch annehmbare Geschmackseigenschaften aufweist, hat die Entwicklung medizinisch annehmbarer flüssiger Arzneimittel zur oralen Verabreichung, insbesondere von Lösungen mit einer ausreichenden Konzentration des aktiven Wirkstoffs, die als Tropfen verabreicht werden können, verhindert. Es besteht daher ein erheblicher Bedarf an einem Derivat des Vincamins, das relativ hohe Löslichkeit in einem pharmazeutisch annehmbaren Lösungsmittel aufweist, aber keine bitteren oder anderweitig unangenehmen Geschmackseigenschaften hat.

Es wurde nun gefunden, daß die neue Verbindung, Vincamin-cyclamat der Formel I, z. B. in Wasser, Glycerin/Ethanol-Gemischen oder in Ethanol/Wasser-Gemischen ausreichend löslich ist, um brauchbare Konzentrationen des aktiven Bestandteils in Lösung zu liefern. Zugleich besitzt Vincamin-cyclamat Geschmackseigenschaften, die für eine Verabreichung als flüssige, orale Arzneiform annehmbar sind. Somit betrifft die Erfindung die neue Verbindung Vincamin-cyclamat, dessen Herstellung sowie auch pharmazeutische Zubereitungen von Vincamin-cyclamat und einem pharmazeutisch annehmbaren Verdünnungsmittel oder Träger. Insbesondere enthalten erfindungsgemäße pharmazeutische Zubereitungen etwa 0,5 bis 10 Gew./Gew.-% Vincamin-cyclamat, gelöst in Wasser oder in einem Lösungsmittelgemisch insbesondere einem aus etwa 10 bis 90 Gewichtsteilen Glycerin und etwa 90 bis 10 Gewichtsteilen Ethanol, oder aus etwa 10 bis 90 Gewichtsteilen Ethanol und etwa 90 bis 10 Gewichtsteilen Wasser. Ein besonders bevorzugtes Arzneimittel enthält etwa 10 Gew./Gew.-% Vincamin-cyclamat, gelöst in einem Gemisch aus etwa 70 Gewichtsteilen Glycerin und etwa 30 Gewichtsteilen Ethanol.

Die erfindungsgemäße neue Verbindung, Vincamin-cyclamat, kann durch Umsetzung von Vincamin-Base und Cyclohexyl-sulfaminsäure in einem geeigneten Lösungsmittel, wie Wasser, niederen Alkoholen, Aceton, Dioxan oder Gemischen dieser Lösungsmittel, bei Temperaturen zwischen 10°C und dem Siedepunkt des betreffenden Lösungsmittels, hergestellt werden. Eine bevorzugte Ausführungsweise besteht darin, daß man die beiden Ausgangsprodukte in der Wärme, in einem der genannten Lösungsmittel löst und das entstandene Vincamin-cyclamat durch Abkühlen ausfällt.

Nach einer anderen Arbeitsweise kann die erfindungsgemäße neue Verbindung auch dadurch hergestellt werden, daß man ein Salz des Vincamins mit einer anorganischen oder organischen Säure, mit einem Alkali- oder Erdalkalisalz der Cyclohexyl-sulfaminsäure zur Reaktion bringt. Die Umsetzung erfolgt entweder durch Lösen von Vincamin-Base in einer anorganischen oder organischen Säure, wie Salzsäure, Schwefelsäure, Phosphorsäure, Salpetersäure, Milchsäure, Citronensäure, Weinsäure, Ketoglutarsäure, Maleinsäure oder unter Einsatz eines vorgeformten entsprechenden Vincamin-salzes, gegebenenfalls unter Zusatz eines geeigneten Lösungsmittels, wie Wasser, niederen Alkohlen, Aceton oder Dioxan und anschließender Zugabe eines Alkali- oder Erdalkalisalzes der Cyclohexyl-sulfaminsäure, vorzugsweise des Natrium- oder Kalium-cyclamates.

Das nach einem der oben beschriebenen Verfahren hergestellte Vincamin-cyclamat kann direkt in der anfallenden Form in pharmazeutischen Zubereitungen eingesetzt werden. Falls gewünscht, ist auch eine weitere Reinigung durch Umkristallisieren aus einem geeigneten Lösungsmittel möglich. Als Lösungsmittel dafür kommen die bei der Herstellung von Vincamin-cyclamat genannten, aber auch andere, dem Fachmann geläufige, in Frage.

Vincamin-cyclamat stellt eine farblose, kristalline Substanz dar, mit der Summenformel $C_{27}H_{39}N_3O_6S$ und dem Molekulargewicht 533,69. Die ermittelten analytischen Daten stehen in Einklang mit der Struktur. Als Beispiel seien die Ergebnisse einer Elementaranalyse aufgeführt:

| | | | | |
|---|---|---|---|---|
| Ber.: | C 60,76 | H 7,36 | N 7,87 | S 6,00%, |
| gef.: | C 60,58 | H 7,32 | N 7,85 | S 6,05%. |

Auch eine Form mit einem Gehalt von 1 mol Kristallwasser ist bekannt. Dieser kommt die Summenformel $C_{27}H_{39}N_3O_6S\ H_2O$ und das Molekulargewicht 551,71 zu. Auch diese Form ist durch analytische Daten belegt. Durch Trocknen der wasserhaltigen Form unter Normaldruck oder im Vakuum bei erhöhter Temperatur, verliert die Substanz das Kristallwasser und geht in die oben charakterisierte, wasserfreie Form über.

Vincamin-cyclamat ist als Gefäßerweiterer zur Behandlung cerebralzirkulatorischer Zustände, wie cerebraler Hypoxie, und damit verbundener neurologischer Störungen, wie Aphasie, Apraxie und Agnosie, in Warmblütern, insbesondere beim Menschen, brauchbar. Das Vincamin-cyclamat wird im allgemeinen oral in Dosen zwischen etwa 0,8 und 1,3 mg/kg/Tag, vorzugsweise etwa 1 mg/kg/Tag, entweder in einer Einzeldosis oder vorzugsweise in zwei bis vier Dosen pro Tag verabreicht.

Während Vincamin-cyclamat, wenn gewünscht, oral in Form fester pharmazeutischer Zubereitungen mit Vincamin-cyclamat und einem pharmazeutisch annehmbaren festen Träger oder Füllstoff, in Dosierungsformen wie Tabletten, Kapseln, Dragees oder Pulvern, verabreicht werden kann, machen es die Löslichkeits- und Geschmackseigenschaften besonders geeignet zur Verabreichung in flüssigen pharmazeutischen Mitteln. Insbesondere pharmazeutische Mittel mit etwa 0,5 bis 10 Gew./Gew.-%, vorzugsweise 10% Vincamin-cyclamat, gelöst in einem Lösungsmittelgemisch aus etwa 10 bis 90 Gewichtsteilen Glycerin und 90 bis 10 Gewichtsteilen Ethanol, oder aus etwa 10 bis 90 Gewichtsteilen Ethanol und etwa 90 bis 10 Gewichtsteilen Wasser. Besonders bevorzugt sind Arzneizubereitungen mit 70 Teilen Glycerin und 30 Teilen Ethanol, da solche Lösungen eine genügende Konzentration des aktiven Bestandteils in Lösung enthalten, um die nötige Dosismenge des Wirkstoffs in einem begrenzten Flüssigkeitsvolumen zu liefern und so leicht oral, insbesondere als Tropfen, verabreicht werden können.

Ethanol für die Verwendung im Lösungsmittelgemisch der oben beschriebenen pharmazeutischen Mittel kann Ethanol von Handelsqualität sein, d. h. 92,3 Gew./Gew.-% Ethanol, Rest Wasser, ist aber bevorzugt absolutes Ethanol (99,9 Gew./Gew.-%). Glycerin für die erfindungsgemäße Verwendung kann 85 Gew./Gew.-% sein, Rest Wasser, ist aber vorzugsweise wasserfreies Glycerin.

Wenn gewünscht, können weitere herkömmliche Bestandteile, wie färbende Mittel oder Farbstoffe, Konservierungsmittel, Geschmacks- bzw. Aromastoffe und dergleichen, den oben beschriebenen pharmazeutischen Mitteln in Lösungsform zugesetzt werden.

Neben den vorstehend beschriebenen pharmazeutischen Mitteln in Lösungsform sind auch solche in flüssiger Form, die Suspensionen von Vincamin-cyclamat in einem pharmazeutisch annehmbaren Medium aufweisen, von Wert für die orale Verabreichung, und zwar auf Grund der überlegenen Geschmackseigenschaften von Vincamin-cyclamat. Solche Suspensionen können etwa 0,5 bis 10 Gew./Gew.-% Vincamin-cyclamat in einem wäßrigen oder organischen Medium, z. B. in wäßrigen Sorbitlösungen, zusammen mit, sofern gewünscht, weiteren Bestandteilen, wie Geschmacks- bzw. Aromamitteln, emulgierenden oder dispergierenden Mitteln, färbendem Material oder Farbstoffen, Konservierungsmittel und dergleichen, enthalten.

Die Erfindung wird durch die nachfolgenden Beispiele weiter erläutert. Natürlich wird sie durch die speziellen Einzelheiten dieser Beispiele nicht eingeschränkt.

## Beispiel 1

0,1 mol (35,4 g) Vincamin-Base und 0,1 mol (17,9 g) Cyclohexyl-sulfaminsäure werden in 800 ml siedendem Wasser gelöst. Man filtriert heiß und kühlt das Filtrat auf 10°C. Die ausgefallenen, nadelförmigen Kristalle werden abgesaugt, mit wenig kaltem Wasser nachgewaschen und bei Raumtemperatur an der Luft getrocknet. Die auf diese Weise hergestellte Form enthält 1 mol Kristallwasser. Durch Trocknen im Vakuum bei 60°C kann daraus die wasserfreie Form erhalten werden. Ausbeute: 40,5 g Vincamin-cyclamat.

Beispiel 2

0,1 mol (35,4 g) Vincamin-Base und 0,1 mol (17,9 g) Cyclohexyl-sulfaminsäure werden in 400 ml eines siedenden Gemisches aus Aceton/Wasser (9 : 1 V/V) gelöst. Man filtriert heiß und kühlt das Filtrat auf 10°C. Der ausgefallene Kristallbrei wird abgesaugt und mit 100 ml Aceton nachgewaschen. Durch Trocknen in Vakuum bei 60°C erhält man 43 g Vincamin-cyclamat.

Beispiel 3

0,1 mol (35,4 g) Vincamin-Base werden in einer auf 80°C erwärmten Mischung aus 500 ml Wasser und 100 ml 1n HCl gelöst. Bei der gleichen Temperatur versetzt man mit einer Lösung von 0,1 mol (20,1 g) Natrium-cyclamat in 300 ml Wasser. Danach wird auf 10°C abgekühlt, der ausgefallene Kristallbrei abgesaugt und mit 200 ml kaltem Wasser nachgewiesen. Nach dem Trocknen bei 60°C im Vakuum erhält man 45 g Vincamin-cyclamat. Bei dem nach dieser Variante hergestellten Produkt ist durch geeignete Methoden auf die Abwesenheit von Chlorid-Ionen zu prüfen.

Beispiel 4

10,0 g Vincamin-cyclamat werden unter Rühren bei Raumtemperatur in 90 g eines Gemischs aus 70 Gewichtsteilen Glycerin (»Europäisches Arzneibuch III«), 85 Gew./Gew.-%, und 30 Gewichtsteilen 99,9%igen Ethanols, gelöst. Nach etwa 4 h wird die Lösung filtriert. Die anfallende Lösung eignet sich für die orale Verabreichung in Form von Tropfen.

**Patentansprüche**

1. Vincamin-cyclamat.
2. Vincamin-cyclamat als pharmazeutischer Wirkstoff.
3. Verfahren zur Herstellung von Vincamin-cyclamat, dadurch gekennzeichnet, daß man

a) Vincamin-Base mit Cyclohexyl-sulfaminsäure in einem geeigneten Lösungsmittel umsetzt, oder
b) ein vorgeformtes oder in situ erzeugtes Vincamin-salz mit einem Alkali- oder Erdalkalisalz der Cyclohexyl-sulfaminsäure umsetzt.

4. Verfahren zur Herstellung von Vincamin-cyclamat nach Anspruch 3, dadurch gekennzeichnet, daß man in Variante a) als Lösungsmittel Wasser, niedere Alkohole, Aceton, Dioxan oder Gemische davon verwendet.
5. Verfahren zur Herstellung von Vincamin-cyclamat nach Anspruch 3b), dadurch gekennzeichnet, daß man als Vincaminsalz ein anorganisches oder organisches Salz der Vincamin-Base und als Alkalisalz der Cyclohexyl-sulfaminsäure Natrium- oder Kalium-cyclamat einsetzt.
6. Arzneimittel enthaltend Vincamin-cyclamat in einem pharmazeutisch annehmbaren Träger oder Verdünnungsmittel.
7. Arzneimittel nach Anspruch 6, enthaltend 0,5 bis 10 Gew./Gew.-% Vincamin-cyclamat, gelöst in einem Lösungsmittel, bestehend aus 10 bis 90 Gewichtsteilen Glycerin und 90 bis 10 Teilen wasserfreiem Ethanol oderr 10 bis 90 Gewichtsteilen Wasser und 90 bis 10 Gewichtsteilen wasserfreiem Ethanol.
8. Arzneimittel nach Anspruch 6, enthaltend 10 Gew./Gew.-% Vincamin-cyclamat, gelöst in einem Lösungsmittel, bestehend aus 70 Teilen Glycerin und 30 Teilen Ethanol.

**Claims**

1. Vincamine-cyclamate.
2. Vincamine-cyclamate as pharmaceutical agent.
3. Process for the production of vincamine-cyclamate, characterized in that

a) a vincamine base is reacted with cyclohexyl sulphamic acid in a suitable solvent, or
b) a preformed vincamine salt or a vincamine salt produced in situ is reacted with an alkali salt or alkaline earth salt of the cyclohexyl sulphamic acid.

4. Process for the production of vincamine-cyclamate according to claim 3, characterized in that in variant a) water, lower alcohols, acetone, dioxan or mixtures thereof are used as solvents.
5. Process for the production of vincamine-cyclamate according to claim 3b, characterized in that an inorganic or organic salt of the vincamine base is used as vincamine salt and sodium cyclamate or

potassium cyclamate as alkali salt of the cyclohexyl sulphamic acid.

6. Drug containing vincamine-cyclamate in a pharmaceutically acceptable carrier or diluent.

7. Drug according to claim 6, containing 0,5 to 10 weight/weight percent vincamine-cyclamate, solved in a solvent, consisting of 10 to 90 parts by weight of glycerine and 90 to 10 parts of water-free ethanol or 10 to 90 parts by weight or water and 90 to 10 parts by weight of water free ethanol.

8. Drug according to claim 6, containing 10 weight/weight percent vincamine-cyclamate, soved in a solvent, consisting of 70 parts of glycerine and 30 parts of ethanol.

**Revendications**

1. Le cyclamate de vincamine.
2. Le cyclamate de vincamine en tant que substance pharmaceutique active.
3. Procédé de préparation de cyclamate de vincamine, caractérisé en ce que

a) on fait réagir de la vincamine base avec de l'acide cyclohexylsulfamique dans un solvant approprié, ou bien
b) on fait réagir un sel de vincamine préalablement formé ou produit in situ avec un sel de métal alcalin ou de métal alcalino-terreux de l'acide cyclohexylsulfamique.

4. Procédé de préparation du cyclamate de vincamine suivant la revendication 3, caractérisé en ce qu'on utilise comme solvant dans la variante a) l'eau, des alcools inférieurs, l'acétone, le dioxanne ou leurs mélanges.

5. Procédé de préparation du cyclamate de vincamine suivant la revendication 3b) caractérisé en ce qu'on utilise comme sel de vincamine un sel inorganique ou organique de la vincamine base et comme sel alcalin de l'acide cyclohexylsulfamique, le cyclamate de sodium ou de potassium.

6. Médicament contenant du cyclamate de vincamine dans un support ou diluant acceptable du point de vue pharmaceutique.

7. Médicament suivant la revendication 6, contenant 0,5 à 10% en poids/poids de cyclamate de vincamine, en solution dans un solvant composé de 10 à 90 parties en poids de glycérine et de 90 à 10 parties d'éthanol anhydre ou de 10 à 90 parties en poids d'eau et de 90 à 10 parties en poids d'éthanol anhydre.

8. Médicament suivant la revendication 6, contenant 10% en poids/poids de cyclamate de vincamine, dissous dans un solvant formé de 70 parties de glycérine et de 30 parties d'éthanol.